# EUROPEAN PATENT APPLICATION

(11) **EP 4 082 618 A1**
(43) Date of publication of application: **02.11.2022**
(21) Application number: 20908009.2
(22) Date of filing: 16.12.2020
(51) Int. Cl.: A61Q 1/00, A61Q 1/02, A61Q 1/04, A61K 8/19, A61K 8/26, A61K 8/58

(54) **COSMETIC**

(30) Priority: 27.12.2019 JP 2019239100
(71) Applicant: Shiseido Company, Ltd., Chuo-ku Tokyo 104-0061 (JP)
(72) Inventor: NAKANO Yusuke, Tokyo 104-0061 (JP)
(74) Representative: Ter Meer Steinmeister & Partner
(86) International application number: PCT/JP2020/047045
(87) International publication number: WO 2021/131987

(57) **Abstract**

An object of the invention is to provide a cosmetic having high chromatic intensity but yet having excellent color fading property. The invention relates to a cosmetic containing α-iron(III) oxide containing 15 mol% or more and 40 mol% or less aluminum.

## Description

### TECHNICAL FIELD

The present invention relates to a cosmetic containing a color material. More specifically, the invention relates to a cosmetic which has high chromatic intensity and does not easily fade in color.

### BACKGROUND ART

A color material is blended in a cosmetic for the purpose of coloring or imparting coating property and plays a role in adding a color of one's taste to the skin or hair and creating healthy and attractive looks.

Color materials blended in cosmetics mainly include synthetic organic colorants (tar colorants), inorganic pigments and natural colorants. As inorganic pigments, oxides, hydroxides, sulfides and silicates of metals and the like are used.

While synthetic organic colorants are generally expected to have property of developing a vivid color, synthetic organic colorants have drawbacks of decomposing and fading in color easily due to light, heat, moisture or the like. On the other hand, because inorganic pigments generally have excellent light resistance and heat resistance and because many thereof do not change in properties even after contacting with solvents or other chemicals, inorganic pigments have excellent color fading property. However, inorganic pigments are considered to be inferior to synthetic organic colorants in vividness (NPL 1).

Accordingly, development of a cosmetic having both property of developing a vivid color and excellent color fading property has been desired.

Here, iron oxide-based red pigments containing aluminum in solid solution have been developed (PTL 1, PTL 2 and NPL 2).

### CITATION LIST

### PATENT LITERATURE

PTL 1: JP-A-2004-43208
PTL2: WO2019/245046

### NON PATENT LITERATURE

NPL 1: "New Cosmetic Science (2nd Edition)", edited by Takeo Mitsui, Nanzando Co., Ltd., 2001
NPL 2: Hideki Hashimoto, "Iron Oxide-Based Red Pigment with Ultra-High Chromatic Intensity", [online], December 4, 2018, New Technology Presentation Meetings [searched on November 19, 2019], internet <URL:https://shingi.jst.go.jp/var/rev0/0000/7855/2018_kogakuin_2.pdf>

### SUMMARY OF INVENTION

### TECHNICAL PROBLEM

PTL 1, PTL 2 and NPL 2 do not disclose that an iron oxide-based red pigment containing aluminum in solid solution is useful as a color material of a cosmetic.

An object of the invention is to provide a cosmetic having high chromatic intensity but yet having excellent color fading property.

### SOLUTION TO PROBLEM

As a result of intensive investigation to achieve the object, the present inventors have found that, using iron oxide that is red iron oxide (α-iron(III) oxide), which is known as an inorganic pigment having a red color, containing a specific amount of aluminum, color vividness which cannot be achieved by conventional red iron oxide can be achieved. The invention has been thus completed.

That is, the invention relates to the following cosmetic.
[1] A cosmetic containing α-iron(III) oxide containing 15 mol% or more and 40 mol% or less aluminum.
[2] The cosmetic described in [1], wherein the α-iron(III) oxide is hydrophobized.
[3] The cosmetic described in [1] or [2], wherein a lightness (L) is 39.5 or more when the α-iron(III) oxide is blended as a single color material.

### ADVANTAGEOUS EFFECTS OF INVENTION

According to the invention, a cosmetic which has excellent color fading property and can achieve property of developing a vivid color can be provided.

### DESCRIPTION OF EMBODIMENTS

### (Color Material)

The cosmetic according to the invention contains α-iron(III) oxide containing 15 mol% or more and 40 mol% or less aluminum (also referred to as "the iron oxide in the invention" below) as a color material.

Because the iron oxide contains 15 mol% or more aluminum, a more vivid color than that of conventional α-iron(III) oxide can be developed while the excellent color fading property that an inorganic pigment has is maintained. A reason is supposed to be that the space for cohesion of primary hematite particles expands and that the reflection wavelength of light changes. It is also supposed that aluminum is doped in the crystal structure of iron oxide in an adequately dispersed state.

The aluminum content is preferably 15 mol% or more, more preferably 20 mol% or more in view of the high chromatic intensity and is preferably 50 mol% or less, more preferably 40 mol% or less in view of maintenance of crystal shape.

The iron oxide in the invention has an average particle size of preferably 0.3 µm to 5 µm and an average thickness of preferably 0.15 µm to 2.5 µm, and the crystal structure thereof is preferably a coin-like structure. Here, the coin-like structure means a cylindrical shape in which the diameter is larger than the thickness.

When the crystal structure of the iron oxide is a coin-like structure, the dispersibility is excellent, and a streak does not easily occur when the iron oxide is blended in foundation, for example.

The iron oxide content of the cosmetic in the invention is preferably 0.05 mass% or more, more preferably 0.075 mass% or more in view of development of an appearance color and is preferably 25 mass% or less, more preferably 20 mass% or less in view of covering property.

As the iron oxide in the invention, for example, those described in Hideki Hashimoto, "Iron Oxide-Based Red Pigment with Ultra-High Chromatic Intensity", [online], December 4, 2018, New Technology Presentation Meetings (NPL 2), WO2019/245046 (PTL 2) and the like can be used.

When the iron oxide in the invention is blended in an oil-based base material, the iron oxide is preferably further hydrophobized (surface-treated). Through the treatment, the dispersibility in the oil-based base material increases, and the chromatic intensity further improves.

The hydrophobization may be treatment which maintains hydrophobicity, and examples thereof include perfluorooctyl triethoxysilane treatment, methicone treatment, dimethicone treatment, hydrogen dimethicone treatment, (alkyl acrylate/dimethicone) copolymer treatment, triethoxycaprylylsilane treatment (OTS treatment), magnesium stearate treatment and the like.

In addition to the essential component, different components which are generally blended in a cosmetic can be blended in the cosmetic of the invention within the scope in which the effects of the invention are not impaired.

The different components include a different color material other than the specific iron oxide, an oil-based component, a component constituting an oil-based base material and different optional components.

### (Different Color Material)

As the different color material, a different color material known as a cosmetic color material can be contained according to the application of the cosmetic.

The different color material can be a synthetic organic colorant selected from a dye, a lake and an organic pigment, a natural colorant, an inorganic pigment selected from an extender pigment, a color pigment (excluding the specific iron oxide) and a white pigment or the like, and one kind thereof or a combination of more than one kind thereof can be used.

### (Oil-Based Component)

The cosmetic of the invention can contain an oil-based component which is generally blended in a cosmetic. Examples of the oil content which can be blended can include the following solid oil contents and liquid oil contents, but the oil-based component is not limited to the examples.

Solid oil contents include solid oils and fats, waxes, hydrocarbons and higher alcohols. Examples thereof include: solid oils and fats such as Japan wax, cocoa butter and hydrogenated castor oil; waxes such as carnauba wax, beeswax, candelilla wax and jojoba wax; hydrocarbon-based waxes such as polyethylene wax, paraffin wax, ceresin and microcrystalline wax; higher alcohols such as behenyl alcohol, cetanol and batyl alcohol; silicon wax: and the like.

Liquid oil contents include: liquid oils and fats such as olive oil, avocado oil, camellia oil, macadamia nut oil, evening primrose oil, jojoba oil, rapeseed oil, egg yolk oil, sesame oil, castor oil, safflower oil, cottonseed oil, soybean oil, tea seed oil, rice bran oil and germ oil; hydrocarbon oils such as squalane and liquid paraffin; ester oils such as isocetyl isostearate, polyglyceryl-2 triisostearate, cetyl 2-ethylhexanoate, 2 heptylundecyl palmitate, diisobutyl adipate, 2-hexyldecyl sebacate, isopropyl myristate, 2-octyldodecyl oleate, diisostearyl malate, ethylene glycol di-2-ethylhexanoate, tri-2-heptylundecanoic acid glyceride, glyceryl diisostearate, glyceryltriisostearate, glyceryl trioctanoate, glyceryl triisopalmitate, trimethylolpropane tri-2-ethylhexanoate, glyceryl tri-2-ethylhexanoate and pentaerythritol tetra-2-ethylhexanoate; chain silicone oils such as dimethylpolysiloxane and methyl phenyl polysiloxane (diphenylsiloxy phenyl trimethicone); cyclic silicone oils such as octamethyl cyclotetrasiloxane and decamethyl cyclopentasiloxane; triglycerin, fluorinemodified oil and the like.

### (Component Constituting Oil-Based Base Material)

The component constituting an oil-based base material can be an oil thickener or a gelling agent.

Examples of the oil thickener (gelling agent) can include dextrin fatty acid esters, glycerin fatty acid esters, organic modified clay minerals and the like. Examples of the dextrin fatty acid esters include dextrin myristate, dextrin palmitate, dextrin (palmitate/2-ethylhexanoate) and the like. Examples of the glycerin fatty acid esters include glyceryl behenate, glyceryl octastearate, glyceryl eicosate and the like.

### (Different Optional Components)

The different optional components include water, UV absorbers, surfactants, antioxidants, fragrance, preservatives, polyhydric alcohols, lower alcohols, volatile oil, inorganic powder, organic powder, active ingredients and the like.

The cosmetic of the invention can be produced according to a method widely used for the conventional cosmetics. That is, the cosmetic can be produced by mixing the specific iron oxide and optional components, stirring and mixing using a homo mixer or the like under heating, pouring into a mold and cooling.

The cosmetic of the invention contains the characteristic iron(III) oxide containing a large amount of aluminum as a color material as described above and thus can achieve high chromatic intensity.

As an indicator expressing a color with values, the L^{∗}a^{∗}b^{∗} color space (JIS Z8729-1994), in which the lightness is expressed by L^{∗} and the chromaticity is expressed by a^{∗} and b^{∗}, is known, and it is regarded that a color is more vivid as the lightness L^{∗} is larger.

When the specific iron oxide in the invention is blended as a single color material, the lightness L^{∗} of the cosmetic of the invention is preferably 39.5 or more, more preferably 40 or more.

The cosmetic of the invention contains the specific iron oxide and thus has both property of developing a vivid color and excellent color fading property. Accordingly, the cosmetic of the invention is particularly suitable for a makeup cosmetic for the purpose of coloring, and examples of the makeup cosmetic include lip cosmetics such as lipsticks and glosses, point makeup cosmetics such as eye shadows and blush, base makeup cosmetics such as foundation, concealer and sunscreen creams and the like.

### EXAMPLES

The invention is explained further specifically referring to Examples below, but the invention is not limited by the Examples. Unless otherwise specified, a blending amount is expressed by mass% based on the total amount.

### (Production of Lipstick Cosmetics)

Lipsticks having the compositions shown in Table 1 below were produced.

Regarding the appearance colors of the lipsticks of the examples, the lightness (L) and the chromaticities (a) and (b) were measured using a sphere benchtop spectrophotometer (X-Rite).

The lipstick of each example was applied onto an arm of an analyst, and the development of the applied color was observed visually. In this regard, using Example 3, which contained only α-iron(III) oxide containing 30 mol% aluminum exhibiting the most vivid applied color among α-iron(III) oxide containing 15 mol% or more and 40 mol% or less aluminum, as the standard for the applied color assessment, the other Examples were assessed.

The color fading property of each lipstick was assessed by (1) the color difference between before and after application of xenon to the lipstick for 50 hours in an atmosphere at 30°C, (2) the color difference between before and after application of a fluorescent light to the lipstick for 300 hours in an atmosphere at 25°C and (3) the color difference four weeks after leaving the lipstick still in an atmosphere at 40°C with humidity of 90%.

**[Table 1]**

| | | Comparative Example | Comparative Example | Example | Example | Example | Example | Comparative Example | Comparative Example | Comparative Example |
|---|---|---|---|---|---|---|---|---|---|---|
| | | 1 | 2 | 1 | 2 | 3 | 4 | 3 | 4 | 5 |
| Polyethylene | | 9 | 9 | 9 | 9 | 9 | 9 | 9 | 9 | 9 |
| Diisostearyl malate | | 20 | 20 | 20 | 20 | 20 | 20 | 20 | 20 | 20 |
| Triethylhexanoin | | 33.9 | 33.9 | 33.9 | 33.9 | 33.9 | 33.9 | 33.9 | 33.9 | 33.9 |
| Polyglyceryl-2 triisostearate | | 20 | 20 | 20 | 20 | 20 | 20 | 20 | 20 | 20 |
| Dimethicone | | 0.05 | 0.05 | 0.05 | 0.05 | 0.05 | 0.05 | 0.05 | 0.05 | 0.05 |
| Tocopherol | | 0.05 | 0.05 | 0.05 | 0.05 | 0.05 | 0.05 | 0.05 | 0.05 | 0.05 |
| Mica | | 12 | 12 | 12 | 12 | 12 | 12 | 12 | 12 | 12 |
| OTS-α-Fe₂O₃ | | 5 | - | - | - | - | - | 0.84 | 0.33 | 0.29 |
| OTS-5 mol% Al-containing α-Fe₂O₃ | | - | 5 | - | - | - | - | - | - | - |
| OTS-15 mol% Al-containing α-Fe₂O₃ | | - | - | 5 | - | - | - | - | - | - |
| OTS-20 mol% Al-containing α-Fe₂O₃ | | - | - | - | 5 | - | - | - | - | - |
| OTS-30 mol% Al-containing α-Fe₂O₃ | | - | - | - | - | 5 | - | - | - | - |
| OTS-40 mol% Al-containing α-Fe₂O₃ | | - | - | - | - | - | 5 | - | - | - |
| Yellow 5 | | - | - | - | - | - | - | 1.25 | 3.34 | 3.53 |
| OTS-yellow iron oxide (Fe(O)OH) | | - | - | - | - | - | - | 2.91 | 1.33 | 1.18 |
| Total | | 100 | 100 | 100 | 100 | 100 | 100 | 100 | 100 | 100 |

| [Table 1 (continued)] | | | | | | | | | | |
|---|---|---|---|---|---|---|---|---|---|---|
| | | Comparative Example | Comparative Example | Example | Example | Example | Example | Comparative Example | Comparative Example | Comparative Example |
| | | 1 | 2 | 1 | 2 | 3 | 4 | 3 | 4 | 5 |
| Appearance color assessment | (L^{∗}) | 36.44 | 40.76 | 42.74 | 42.82 | 42.24 | 44.28 | 46.63 | 44.52 | 44.33 |
| | (a^{∗}) | 25.84 | 28.61 | 30.59 | 30.61 | 29.69 | 28.02 | 25.94 | 33.78 | 34.48 |
| | (b^{∗}) | 14.24 | 21.49 | 25.59 | 25.95 | 25.85 | 24.04 | 27.1 | 29.32 | 29.4 |
| Applied color assessment | Redness | +3 | +2 | +1 | +1 | ±0 | -0.5 | 0 | +1 | +0.5 |
| | Yellow | -3 | -2 | ±0 | ±0 | ±0 | +0.5 | +1 | ±0 | -0.5 |
| | Blue | 3 | 2 | ±0 | ±0 | ±0 | ±0 | ±0 | ±0 | ±0 |
| | Whiteness | -1 | -1 | ±0 | ±0 | ±0 | ±0 | +0.5 | +0.5 | ±0 |
| | Vividness | -3 | -2 | -0.5 | -0.5 | ±0 | ±0 | -2 | +1 | ±0 |
| | Transparency (free of dullness) | -3 | -1 | -0.5 | -0.5 | ±0 | -0.5 | -1 | -0.5 | ±0 |
| Light resistance assessment (xenon application, 30°C, 50 hours) ΔE | | 0.31 | 0.23 | 0.47 | 0.68 | 0.40 | 0.79 | 1.87 | 4.46 | 4.41 |
| Light resistance assessment (fluorescent light application, 300 hours) ΔE | | 0.28 | 0.10 | 0.25 | 0.61 | 0.25 | 0.72 | 2.91 | 6.43 | 6.60 |
| Moisture color change assessment (40°C 90% moisturizing, 4 weeks) ΔE | | 0.08 | 0.67 | 0.25 | 0.44 | 0.42 | 0.74 | 1.25 | 0.65 | 0.83 |

The results of the appearance color assessment show that the lipsticks of Examples 1 to 4, which contained iron oxide containing a specific amount of aluminum, had lightness (L) of 40 or more and had excellent color developing property.

Moreover, from the results of the applied color assessment, while the lipstick of Comparative Example 1, in which conventional red iron oxide containing no aluminum was blended, and the lipstick of Comparative Example 2, in which iron oxide having a small aluminum content was used as a color material, exhibited a reddish brown color, the lipsticks of Examples 1 to 4, in which iron oxide containing a specific amount of aluminum was blended, exhibited a vivid orange color comparable to that of the lipsticks of Comparative Examples 3 to 5 using an organic color material.

Furthermore, regarding the color fading property, although the color differences ΔE of the lipsticks of Comparative Examples 3 to 5 using an organic color material changed largely in all the tests, the changes in the color differences of the lipsticks of Examples 1 to 4 using α-iron oxide were small.

### (Formulation Examples)

Formulation examples of foundation and a concealer are shown below.

**[Table 2]**

| Formulation Example, liquid foundation | (mass%) |
|---|---|
| Cyclopentasiloxane | 40 |
| Isopropyl myristate | 3 |
| PEG-10 dimethicone | 2.5 |
| Polyglyceryl-2 diisostearate | 1 |
| (PEG-15/lauryl polydimethylsiloxyethyl dimethicone) crosspolymer | 5 |
| Disteardimonium hectorite | 0.8 |
| Hydrophobized titanium oxide microparticles | 8 |
| OTS-treated titanium dioxide | 2 |
| OTS-treated 30 mol% Al-containing α-Fe₂O₃ | 0.35 |
| OTS-treated yellow iron oxide | 1.1 |
| OTS-treated black iron oxide | 0.05 |
| Ion exchanged water | Balance |
| Glycerin | 3.5 |
| Ethanol | 3.5 |
| Phenoxyethanol | 0.5 |

**[Table 3]**

| Formulation Example, concealer | (mass%) |
|---|---|
| Cyclopentasiloxane | 40 |
| Isopropyl myristate | 3 |
| PEG-10 dimethicone | 2.5 |
| Polyglyceryl-2 diisostearate | 1 |
| (PEG-15/lauryl polydimethylsiloxyethyl dimethicone) crosspolymer | 5 |
| Disteardimonium hectorite | 0.8 |
| Hydrophobized titanium oxide microparticles | 8 |
| OTS-treated titanium dioxide | 25 |
| OTS-treated 30 mol% Al-containing α-Fe₂O₃ | 0.35 |
| OTS-treated yellow iron oxide | 1.1 |
| OTS-treated black iron oxide | 0.05 |
| Ion exchanged water | Balance |
| Glycerin | 3.5 |
| Ethanol | 3.5 |
| Phenoxyethanol | 0.5 |

**[Table 4]**

| Formulation Example, solid foundation | (mass%) |
|---|---|
| Cyclopentasiloxane | 35 |
| Isopropyl myristate | 3 |
| PEG-10 dimethicone | 2.5 |
| Polyglyceryl-2 diisostearate | 1 |
| (PEG-15/lauryl polydimethylsiloxyethyl dimethicone) crosspolymer | 5 |
| Disteardimonium hectorite | 0.8 |
| Microcrystalline wax | 2 |
| Polyethylene wax | 3 |
| Hydrophobized titanium oxide microparticles | 8 |
| OTS-treated titanium dioxide | 7 |
| OTS-treated 30 mol% Al-containing α-Fe₂O₃ | 0.35 |
| OTS-treated yellow iron oxide | 1.1 |
| OTS-treated black iron oxide | 0.05 |
| Ion exchanged water | Balance |
| Glycerin | 3.5 |
| Ethanol | 3.5 |
| Phenoxyethanol | 0.5 |

Although the invention has been explained in detail referring to specific embodiments, it is obvious to one skilled in the art that various changes and modifications can be added without departing from the spirit and the scope of the invention. The present application is based on a Japanese patent application filed on December 27, 2019 (patent application No. 2019-239100), and the contents thereof are incorporated here by reference.

## Claims

1. A cosmetic containing α-iron(III) oxide containing 15 mol% or more and 40 mol% or less aluminum.

2. The cosmetic according to claim 1, wherein the α-iron(III) oxide is hydrophobized.

3. The cosmetic according to claim 1 or 2, wherein a lightness (L) is 39.5 or more when the α-iron(III) oxide is blended as a single color material.
